# EUROPEAN PATENT SPECIFICATION

(11) **EP 1 504 736 B1**
(45) Date of publication and mention of the grant of the patent: **06.09.2006**
(21) Application number: 04103677.3
(22) Date of filing: 30.07.2004
(51) Int. Cl.: A61F 5/01

(54) **Adjustable walker brace and hinge for such walker brace**
Einstellbare Gehstütze und Scharnier für eine solche Stütze
Support pour promeneur réglable et charnière pour un tel support

(30) Priority: 06.08.2003 IT VR20030098
(43) Date of publication of application: 09.02.2005
(73) Proprietor: F.G.P. SRL, 37062 Dossobuono (IT)
(72) Inventor: Turrini, Alberto, 37060 Castel d'Azzano (VR) (IT); Ferrigolo, Moreno, 37062 Dossobuono (VR) (IT)
(74) Representative: Petraz, Gilberto Luigi

(56) References cited:
- EP-A- 1 086 672
- US-A- 5 672 152
- US-A- 5 814 000
- US-A- 5 938 629
- US-B1- 6 203 511

## Description

This invention concerns a walker brace for the foot and/or the ankle.

More specifically, this invention refers to an arch support walker or brace advantageously presenting a structure made from rigid material, for example plastic or lightweight metal alloy, with appropriate articulated joints at the level of the ankle and with a frame that can be used for treatment following contusion and/or sprains involving the foot, the heel and/or the ankle.

This invention can be applied in the medical sector with particular reference to manufacturers of walkers or braces.

### BACKGROUND ART

The use of walkers or braces is known for patients suffering from fractures or sprains in the arch of the foot in parts presenting particularly complex bone structures.

The trauma often involves difficulty in walking due to the pain felt on placing the foot on the ground; in particular, if the trauma consists of a fracture, it is necessary to immobilise the injured part to ensure subsequent self-reconstruction of the bone structure to be knitted together.

For a period of time proportional to the severity and extent of the fracture, the patient must keep the affected area practically immobile and must therefore wear an arch support walker, generally consisting of plaster casts supported by a metal frame designed to guarantee secure immobility of the injured part.

The walker or brace involves the entire plantar area, affecting the foot and the heel and forcing the patient to use the tibial portion of the leg.

This condition creates considerable difficulties for the patient who, having to walk with minimal support pressure, is obliged to carry out unusual movements that are also difficult to perform.

Above all, background art walkers are difficult to adjust as regards the tension of the bindings and the extension of the immobilised part.

For the comfort of the user and reliable immobilisation of the injured part, an arch support walker must ensure uniform pressure on the foot also in order to guarantee correct blood flow in the immobilised area.

A number of solutions have already been proposed, see for example the document US-A-3955565 which envisages the use of an orthopedic walker consisting of two half-shells made from thin rigid plastic material. This walker is boot-shaped, presenting a front half-shell that can be adapted to the tibial area and to the upper part of the foot and a rear half-shell coming into contact with the heel, the sole of the foot and the calf.

In US-B1-6203511, there is disclosed an orthopaedic joint comprising a crown, two rotatable disks with slots involving a predefined circumferential portion of the disk, the slots being suited to accommodate a pawl, and means for temporary blocking the disk to the crown.

One drawback of the walkers currently available is represented by the fact that they are particularly cumbersome as well as being difficult to fix to the part to be protected.

Another drawback is that these walkers are unlikely to provide uniform pressure over the entire injured area, causing situations of neurological and vascular suffering which can, in the long-term, lead to superficial wounds.

A further drawback is represented by the fact that currently available arch support walkers are extremely inflexible as they are difficult to adjust. This refers to the possible angular movements of the foot with respect to the tibia in relation to the trauma suffered by the patient.

### DISCLOSURE OF THE INVENTION

This invention proposes to provide an arch support walker for the foot and/or the ankle, able to eliminate or significantly reduce the drawbacks described above.

This invention also proposes to provide a walker that is easy to produce as well as being reliable and secure.

This is achieved by means of an arch support walker for the foot and/or the ankle with the features described in the main claim.

The dependent claims describe advantageous embodiments of the invention.

The arch support walker according to the invention comprises a shell equipped with a concave inner portion, each longitudinal edge of which presents a wing designed to be fixed to a corresponding hinged joint, in order to restrain a respective upright free to carry out angular movements within an adjustable range.

According to the invention, each joint comprises at least one crown, restrained to the respective wing by any appropriate fixing means.

Each crown is deigned to accommodate a disc with a slot and equipped with means for the temporary blocking of the crown.

These temporary blocking means consist of a pair of radial cursors, slidingly accommodated in a respective groove in the disc, elastically pushed towards internal teeth of the crown so as to engage with the teeth and prevent the angular oscillation of the disc with respect to the crown.

The end of each cursor, projecting from the joint, is equipped with a pushbutton which can be easily pressed from the outside to disengage the cursor from the inner teeth and release the disc from the crown.

The slot in the disc involves a predefined circumferential portion and is designed to accommodate a respective pawl of the upright in order to limit the angular movements.

A different positioning of the disc with respect to the crown, while not changing the oscillation range of the upright, allows a different maximum and minimum angle of inclination of the upright with respect to the shell.

This invention envisages the use of an articulated joint comprising two crowns, one on top of the other, with the slots in the disc defining a through opening of adjustable width in relation to the position of the cursors.

By means of the independent adjustment of the crowns, the permitted angular movement of the uprights can vary both in intermediate value and in width, in relation to the motor possibilities of the patient.

Repositioning of the cursors is particularly easy and can be performed directly by the user. Thanks to a slight pressure on the cursor pushbuttons, it is possible to overcome the contrasting action of the means of elastic loading and disengage the cursor from the inner teeth of the crown, releasing the respective disc and allowing it to oscillate with respect to the crown. When the required position has been reached, releasing the pushbuttons causes them to be pushed towards the teeth and engage in the new position.

Both the shell and the uprights are equipped with slits and/or holes designed to accommodate appropriate elements for fixing the walker to the patient's foot.

The walker can for example be made from rigid composite moulded plastic which is non-allergic and/or from lightweight metal alloy.

### DESCRIPTION OF THE DRAWINGS

Other features and advantages of the invention will become evident on reading the description given below of one embodiment of the invention, given as a non-binding example, with the help of the attached drawings, in which:
- figure 1 shows a perspective view, slightly from above, of a walker according to the invention;
- figure 2 shows a view similar to figure 1 but with the articulated joint presenting parts in cross-section;
- figure 3 is a front elevated enlarged-scale view of an articulated joint;
- figure 4 shows a rear view of the joint shown in figure 3;
- figure 5 represents a perspective view, slightly from above and exploded, of a joint; and
- figure 6 shows a view similar to figure 4 but with a joint consisting of two crowns.
- figure 7 is a perspective front view of the walker shown in figure 1.

### DESCRIPTION OF ONE EMBODIMENT

In the figures, the reference number 10 indicates in general a walker, in the case in question an arch support walker 10 for the foot and/or the ankle.

The walker 10 comprises a shell 11 with an inner concave portion 12 designed to accommodate the patient's foot.

The front part of the shell 11 is fairly shallow, gradually increasing in depth towards the central part of the walker 10.

Close to its rear part, each longitudinal side edge 13 of the shell 11 presents a wing 14 on which a respective articulated joint 15 is fixed and which acts as a hinge to restrain a corresponding upright 16.

The part of each wing 14 facing the rear portion of the shell 11 presents a slit 17 designed to accommodate means for fixing the walker 10 to the patient's foot.

Each upright 16 consists of a pair of strips 18 which at their free end present holes 19 designed to accommodate means for fixing the walker 10 to the patient's foot.

The circular longitudinal end 20 of each strip 18, fitted on top of the respective joint 15, has a central pin 21 and a pawl 22 protruding towards the joint 15 and close to the edge of the upright 16.

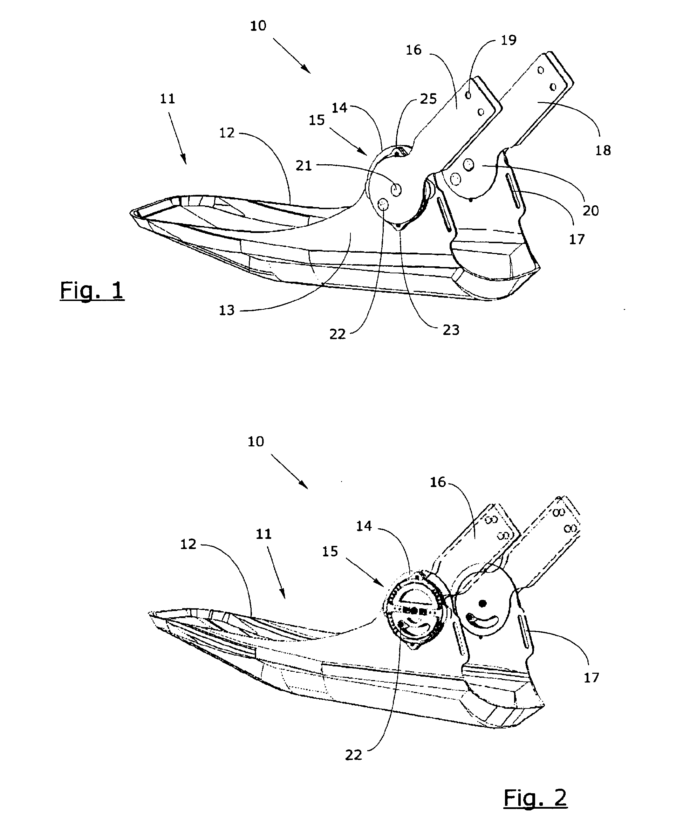

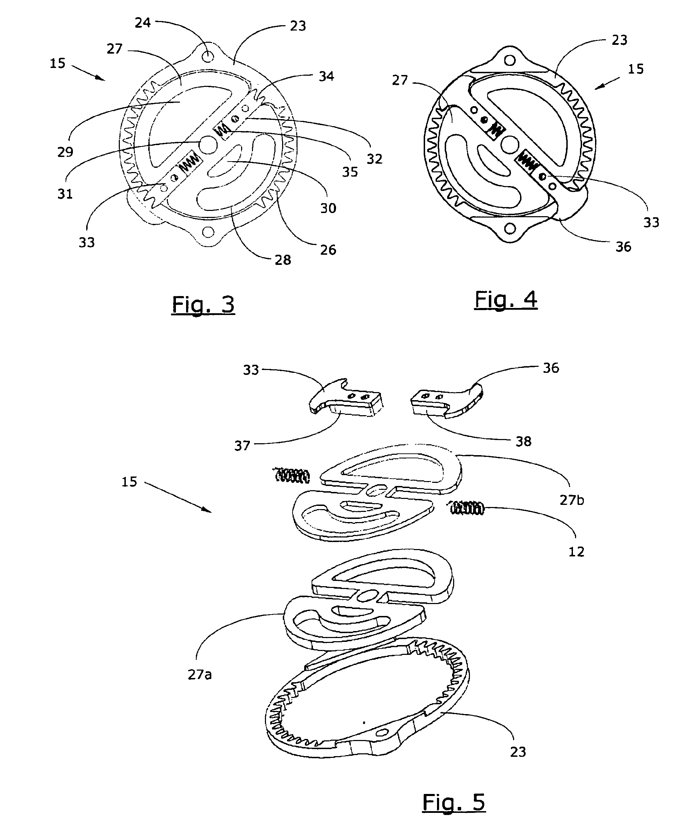

As can be seen in figures 2 and 3, each joint 15 comprises at least one crown 23 equipped with through holes 24 designed to accommodate fixing means, which can consist of screws 25, to attach the joint 15 to the respective wing 13.

Most of the inner part of the crown 23 is equipped with teeth 26, and is designed to accommodate a disc 27 presenting a slot 28 in a limited circumferential portion.

The disc 27 presents a wide cavity 29 lightening the diametral portion opposite the slot 28, a small opening 30 close to the slot 28, a central hole 31, designed to accommodate the pin 21, and a pair of grooves 32 lying on the same diameter.

Each groove 32 is designed to accommodate a respective cursor 33 equipped with teeth 34 facing the outer part of the disc 27.

The bottom end of each groove 32, close to the central hole 31, accommodates a means of elastic loading, for example a helical spring 35, designed to strike against the respective cursor 33.

With reference to figure 4, it can be noted that the end of each cursor 33, facing the outer part of the disc 27, presents a pushbutton 36 surmounting the teeth 34.

As can be seen in figure 5, the disc 27 consists of a lower half-portion 27a and an upper half-portion 27b with the respective grooves 32b being wider than the grooves 32a in the lower half-portion 27a.

Each cursor 33 is equipped with a plinth 37, slidingly accommodated in the respective groove 32a, surmounted by a platform 38 terminating with the pushbutton 36 and designed to be slidingly accommodated in the respective groove 32b.

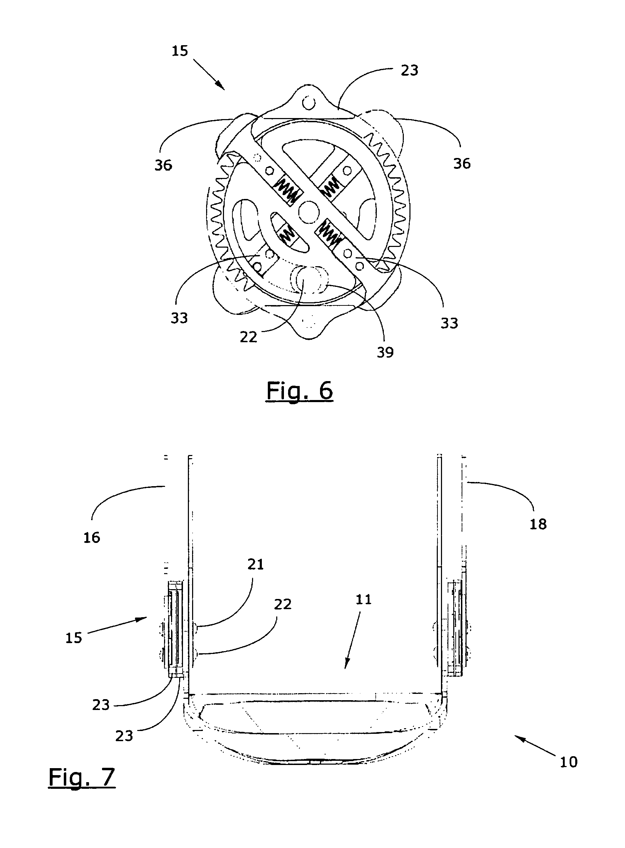

With reference to figure 6, a joint 15 according to the invention foresees two crowns 23, placed one on top of the other and with a common pin 21, enclosing the respective discs 27 which can be differently positioned in order to define a space 39, between the slots 27, designed to accommodate the pawl 22 of the upright 16.

According to this particularly advantageous embodiment of the invention, the position assigned by the slot 28 of one disc 27 guarantees a range of angular positions for the pawl 22, and consequently for the respective upright 16, while the position of the other disc 27 ensures an even more limited and adjustable movement of the pawl 22.

The assembly of the two crowns 23 with the shell 11 and the respective uprights can be clearly seen in the representation of the walker according to the invention shown in figure 7.

In the rest position, each cursor 33 is pushed radially towards the exterior of the disc 27 with its teeth 34 engaging with the teeth 26 of the crown 23.

The angular positioning of each disc 27 with respect to the corresponding crown 23 is easily performed by pressing the diametrally opposite pushbuttons 36 to overcome the contrasting action of the springs 35 and moving the disc 27 to the required position. Subsequent releasing of the pushbuttons 36 allows a new engagement of the teeth 34 in the teeth 26, producing temporary blocking of the disc 27 with respect to the corresponding crown 23.

The shell 11 and the uprights 16 can be made, for example, from rigid non-allergic composite plastic and/or from lightweight metal alloy.

The joints 15 can be made from rigid composite plastic and/or from lightweight metal alloy.

The invention is described above with reference to a preferential embodiment.

The invention is nevertheless susceptible to numerous variations within the framework of technical equivalents.

## Claims

1. An articulated joint (15) for an arch support walker (10), comprising at least one crown (23) designed to accommodate a respective disc (27) equipped with a slot (28) suited to accommodate a pawl (22), the slot involving a predefined circumferential portion of the disc (27), the disc (27) being provided with means of temporary blocking to the crown (23), **characterised in that** the temporary blocking means consist of a pair of radial cursors (33), slidingly housed in respective grooves (32) of the disc (27), and **in that** each cursor (33) is equipped with means of elastic loading (35) designed to push it towards the exterior of the disc (27).

2. A joint (15) according to claim 1, **characterised in that** each cursor (33) presents teeth (34) facing the exterior of the disc (27) and **in that** the crown (23) is provided with corresponding inner teeth (26).

3. A joint (15) according to any of the foregoing claims, **characterised in that** the end of each cursor (33), protruding from the joint (15), is equipped with a pushbutton (36).

4. A joint (15) according to any of the foregoing claims, **characterised in that** it comprises two crowns (23), one on top of the other, and **in that** the slots (28) of the discs (27) define a through opening (39) adjustable in width.

5. A joint (15) according to any of the foregoing claims, **characterised in that** the joints (15) are made from lightweight metal alloy.

6. An arch support walker (10) comprising a shell (11) with an inner concave portion (12), each longitudinal edge (12) of which presents a wing (13), **characterised in that** the wing (13) is fixed to a corresponding hinged joint (15) according to any of the claims from 1 to 6, designed to restrain a respective upright (16) free to carry out angular movements within an adjustable range.

7. A walker (10) according to claim 6, **characterised in that** the longitudinal end of each upright (16), close to the joint (15), is equipped with a pawl (22) designed to be accommodated in the slot (28) and/or in the through opening (39).

8. A walker (10) according to claim 6 or 7, **characterised in that** the shell (11) and the uprights (16) are equipped with slits (17) and/or holes (19) designed to accommodate appropriate means for fixing the walker (10) to the patient's foot.

9. A walker (10) according to any of the foregoing claims from 6 to 8, **characterised in that** it is made from rigid non-allergic composite plastic.

## Patentansprüche

1. Gegliedertes Gelenk (15) für eine das Fußgewölbe stützende Gehhilfe (10), bestehend aus mindestens einem Kranz (23), der dazu dient, eine dazugehörige Scheibe (27) aufzunehmen, die in einem vorgegebenen Umfangsbereich der Scheibe (27) mit einem Schlitz (28) versehen ist, der geeignet ist, eine Klinke (22) aufzunehmen, wobei die Scheibe (27) eine Vorrichtung aufweist, welche den Kranz (23) vorläufig blockiert.
**dadurch gekennzeichnet, dass**
das vorläufige Blockiermittel aus einem Paar radialer Schieber (33) besteht, die gleitend in dazugehörigen Vertiefungen (32)der Scheibe (27) untergebracht sind, und
dass jeder Schieber (33) ein Federbelastungsmittel (35) aufweist, das dazu dient, den Schieber in Richtung Außenfläche der Scheibe (27) zu drücken.

2. Gelenk (15) gemäß Anspruch 1, **dadurch gekennzeichnet, dass** jeder Schieber (33) Zähne (34) aufweist, die sich dem Außenteil der Scheibe (27) zuwenden, und dass der Kranz (23) mit entsprechenden inneren Zähnen (26) versehen ist.

3. Gelenk (15) gemäß einem der vorhergehenden Ansprüche, **dadurch gekennzeichnet, dass** das aus dem Gelenk (15) herausragende Ende jedes Schiebers (33) mit einer Drucktaste (36) versehen ist.

4. Gelenk (15) gemäß einem der vorhergehenden Ansprüche, **dadurch gekennzeichnet, dass** es zwei übereinander liegende Kränze (23) aufweist und das die Schlitze (28) der Scheiben (27) eine Durchlassöffnung (39) darstellen, deren Breite verstellbar ist.

5. Gelenk (15) gemäß einem der vorhergehenden Ansprüche, **dadurch gekennzeichnet, dass** die Gelenke (15) aus einer Leichtmetalllegierung hergestellt sind.

6. Eine das Fußgewölbe stützende Gehhilfe (10), die aus einer einen inneren konkaven Bereich (12) aufweisenden Schale (11) besteht, deren Längsflanken (13) jeweils einen Flügel (14) aufweisen, der an einem dazugehörigen Scharniergelenk (15) befestigt ist, gemäß einem der Ansprüche 1 bis 5, das dazu dient, ein dazugehöriges Vertikalteil (16) festzuhalten, das in der Lage ist, innerhalb eines verstellbaren Bereichs Winkelbewegungen auszuführen.

7. Gehhilfe (10) gemäß Anspruch 6, **dadurch gekennzeichnet, dass** das Längsende jedes Vertikalteils (16) in Nähe des Gelenks (15) eine Klinke (22) aufweist, die so beschaffen ist, dass sie in Schlitz (28) und/oder in der Durchlassöffnung (39) aufgenommen werden kann.

8. Gehhilfe (10) gemäß Anspruch 6 oder 7, **dadurch gekennzeichnet, dass** die Schale (11) und die Vertikalteile (16) Schlitze (17) und/oder Löcher (19) aufweisen, die so beschaffen sind, dass sie eine geeignete Vorrichtung zur Befestigung der Gehhilfe (10) am Fuß des Patienten aufnehmen können.

9. Gehhilfe (10) gemäß einem der Ansprüche 6 bis 8, **dadurch gekennzeichnet, dass** sie aus einem starren, antiallergischen Plastikverbundstoff hergestellt ist.

## Revendications

1. Joint articulé (15) pour dispositif de support de voûte plantaire pendant la marche (10), comprenant au moins une couronne (23) conçue pour loger un disque respectif (27) équipé d'une fente (28) permettant de loger un doigt (22), la fente impliquant une portion circonférentielle prédéfinie du disque (27), le disque étant pourvu de moyens de blocage temporaires par rapport à la couronne (23), **caractérisé en ce que** les moyens de blocage temporaires se composent d'une paire de curseurs radiaux (33), logés de manière coulissante dans des rainures respectives (32) du disque (27), et **en ce que** chaque curseur (33) est équipé de moyens de contrainte élastiques (35) conçus pour le repousser vers l'extérieur du disque (27).

2. Joint (15) selon la revendication 1, **caractérisé en ce que** chaque curseur (33) présente des dents (34) faisant face à l'extérieur du disque (27) et **en ce que** la couronne (23) est pourvue de dents internes correspondantes (26).

3. Joint (15) selon l'une quelconque des revendications précédentes, **caractérisé en ce que** l'extrémité de chaque curseur (33), faisant saillie à partir du joint (15), est équipée d'un bouton-poussoir (36).

4. Joint (15) selon l'une quelconque des revendications précédentes, **caractérisé en ce qu'**il comporte deux couronnes (23), l'une au-dessus de l'autre, et **en ce que** les fentes (28) des disques (27) définissent une ouverture traversante (39) réglable en largeur.

5. Joint (15) selon l'une quelconque des revendications précédentes, **caractérisé en ce que** les joints (15) sont réalisés dans un alliage métallique léger.

6. Dispositif de support pour voûte plantaire pendant la marche (10), comprenant une coquille (11) avec une portion concave interne (12), dont chaque bord longitudinal (12) présente une aile (13), **caractérisé en ce que** l'aile (13) est fixée à un joint articulé correspondant (15) selon l'une quelconque des revendications 1 à 6, conçu pour limiter un montant (16) respectif d'exécuter librement des mouvements angulaires dans une plage réglable.

7. Dispositif (10) selon la revendication 6, **caractérisé en ce que** l'extrémité longitudinale de chaque montant droit (16), proche du joint (15), est équipée d'un doigt (22) conçu pour se loger dans la fente (28) et/ou dans l'ouverture traversante (39).

8. Dispositif (10) selon la revendication 6 ou 7, **caractérisé en ce que** la coquille (11) et les montants (16) sont équipés de rainures (17) et/ou de trous (19) conçus pour loger des moyens appropriés permettant de fixer le dispositif (10) au pied du patient.

9. Dispositif (10) selon l'une quelconque des revendications précédentes 6 à 8, **caractérisé en ce qu'**il est réalisé dans un plastique composite non allergique rigide.
